# EUROPÄISCHE PATENTANMELDUNG

(11) **EP 0 860 171 A2**
(43) Veröffentlichungstag der Anmeldung: **26.08.1998**
(21) Anmeldenummer: 98102405.2
(22) Anmeldetag: 12.02.1998
(51) Int. Cl.: A61L 27/00

(54) **Bioresorbierbare wachsartige Zusammensetzung, ihre Verwendung als Knochenwachs und Verfahren zur Herstellung**

(30) Priorität: 20.02.1997 DE 19706621
(71) Anmelder: DEUTSCHE INSTITUTE FÜR TEXTIL- UND FASERFORSCHUNG STUTTGART Stiftung des öffentlichen Rechts, 73770 Denkendorf (DE)
(72) Erfinder: Oberhoffner, Sven, Dr., 71384 Weinstadt-Benzach (DE); Planck, Heinrich, Prof. Dr., 72622 Nürtingen (DE)
(74) Vertreter: Patentanwälte Ruff, Beier, Schöndorf und Mütschele

(57) **Zusammenfassung**

Die Erfindung betrifft eine bioresorbierbare wachsartige Zusammensetzung, die im wesentlichen aus einem statistischen Terpolymer mit völlig amorpher Struktur gebildet ist. Das Terpolymer wird durch statistische Copolymerisation aus Trimethylencarbonat, Caprolacton und Glykolid gebildet. Die Zusammensetzung ist zur Herstellung eines Knochenwachses verwendbar, das ganz oder teilweise aus der Zusammensetzung gebildet ist.

## Beschreibung

Die vorliegende Erfindung betrifft eine bioresorbierbare wachsartige Zusammensetzung, ihre Verwendung als Knochenwachs und Verfahren zu ihrer Herstellung.

In der Medizin und insbesondere in der Chirurgie stellt sich bei Eingriffen an Knochen das Problem der Hämorrhagie an angeschnittenen Knochenflächen. Um diese Blutungen aus Knochen zu unterdrücken, wurden hämostatische Zusammensetzungen entwickelt, die auf die Knochendefekte aufgebracht bzw. in Vertiefungen wie Bohrlöcher eingebracht werden. Diese Zusammensetzungen werden allgemein als Knochenwachs oder Knochensiegel bezeichnet.

Materialien zur Auffüllung von Knochendefekten müssen bei Raumtemperatur leicht plastisch verformbar sein und eine gute Verbindung zum Knochengewebe aufweisen. Die auf dem Markt befindlichen und in Veröffentlichungen beschriebenen Produkte sind unterteilbar in nicht resorbierbare und resorbierbare Materialien. Zu den ersten gehört gereinigtes Bienenwachs, das teilweise mit Additiven (z. B. Vaseline) versehen wird, um die Plastizität zu verbessern. Auch Ethylenoxid-Propylenoxid-Copolymere (US 4439420, Ethicon) und niedermolekulare Ethylen-Copolymer-Wachse (US 3395217, Dow-Chemicals) sind dieser Klasse zuzuordnen. Mitunter werden absorbierbare Zusatzstoffe wie beispielsweise Mandelöl, Sojaöl, Lebertran oder Olivenöl zugesetzt. Als Adhäsionsmittel dienen Zusatzstoffe wie Polyglucoside, Gelatine, Collagen-Gel, Stärke, Zucker und Cellulosederivate, um nur einige als Beispiel zu nennen.

Im Bereich der resorbierbaren Materialien zur Haemostase bei Knochendefekten werden im Stand der Technik Mischungen aus Fibrin- und Collagen-Pulver in einer wasserlöslichen Grundsubstanz (DE 2852319, Ethicon), Polymerisate aus Diglykolsäure mit polyfunktionellen Alkoholen (EP 722966, Ethicon) und Mischungen aus Oligomeren des D- oder L-Laktids mit amorphen, auf Milchsäure basierenden Oligomeren (DE 4235312, Boehringer) eingesetzt. In den europäischen Patenten von Henkel (EP 3525888, EP 3229540 und EP 290983) werden Systeme aus halbfesten Hydroxycarbonsäure-Oligomeren, speziell solche aus Milch- und Glykolsäure beschrieben, die in Gegenwart eines Molekulargewichtsreglers wie beispielsweise Glycerin hergestellt werden.

Auch den resorbierbaren Knochenwachsen werden teilweise die oben genannten Additive zur Verbesserung der Plastifizierbarkeit sowie der Adhäsion gegenüber dem Knochengewebe zugesetzt (siehe US 444078 von Ethicon und EP 572272 A1 von Johnson & Johnson Medical Inc.). Zur Vermeidung einer pH-Absenkung durch die Degradation wird beispielswiese eine phosphatgepufferte Kochsalzlösung beigemischt (siehe EP 572272 A1 von Johnson & Johnson Medical Inc.).

Ein Problem bei bekannten Knochenwachsen ist die Entzündung des angrenzenden Gewebes und damit verbunden eine Verzögerung bzw. Verlangsamung des regenerierenden Knochenwachstums. Besonders nicht resorbierbare Produkte wie Bienenwachs können neben den Entzündungserscheinungen auch als mechanische Barriere wirken, die eine Schließung der Defekte durch Gewebewachstum verhindern. Auch können sie eine chronische Fremdkörperreaktion hervorrufen und es können für den Patienten gefährliche Wachs-Embolien auftreten.

Die Erfindung stellt sich die Aufgabe, ein resorbierbares synthetisches Polymer zur Verfügung zu stellen, das einfach und zuverlässig als Knochenwachs zu verwenden ist, dabei die Probleme der Knochenwachse aus dem Stand der Technik vermeidet, und das einfach und kostengünstig herstellbar ist.

Diese Aufgabe wird gelöst durch eine bioresorbierbare wachsartige Zusammensetzung, die im wesentlichen aus einem statistischen Terpolymer mit völlig amorpher Struktur gebildet ist. In einer besonderen Ausführungsform der Erfindung kann die bioresorbierbare wachsartige Zusammensetzung vorzugsweise vollständig aus Terpolymer gebildet sein.

Das erfindungsgemäße Terpolymer kann sich dadurch auszeichnen, daß es aus Trimethylencarbonat, ε-Caprolacton und Glykolid gebildet ist. Anstelle des dimeren Glykolids kann auch Glykolsäure verwendet werden, was eine Regulierung des Molekulargewichts erlaubt.

Insbesondere kann Trimethylencarbonat in einem Anteil von 5 bis 70 Gew.-%, ε-Caprolacton in einem Anteil von 5 bis 70 Gew.-% und Glykolid in einem Anteil von 10 bis 60 Gew.-% im erfindungsgemäßen Terpolymer enthalten sein. Die Gewichtsanteile der Komponenten Trimethylencarbonat, ε-Caprolacton und Glykolid sind so gewählt, daß sie zusammengenommen 100 Gew.-% des Terpolymers ausmachen. Erfindungsgemäß bevorzugt kann im Terpolymer Trimethylencarbonat insbesondere in einem Anteil von 10 bis 40 Gew.-%, ε-Caprolacton insbesondere in einem Anteil von 10 bis 40 Gew.-% und Glykolid insbesondere in einem Anteil von 20 bis 40 Gew.-% enthalten sein.

Im Terpolymer gemäß der vorliegenden Erfindung können Trimethylencarbonat und ε-Caprolacton in einem Gewichtsverhältnis zwischen 80 : 20 und 20 : 80, insbesondere in einem Verhältnis zwischen 70 : 30 und 30 : 70 vorhanden sein. Bevorzugt können im Terpolymer Trimethylencarbonat und ε-Caprolacton in einem Gewichtsverhältnis von 50 : 50 vorhanden sein. In einer anderen Ausführungsform kann im Terpolymer ε-Caprolacton in einem höheren Anteil als Trimethylencarbonat vorhanden sein.

Das Terpolymer gemäß der vorliegenden Erfindung kann sich mit Vorteil dadurch auszeichnen, daß es durch statistische Copolymerisation von Trimethylencarbonat, ε-Caprolacton und Glykolid hergestellt ist.

Bevorzugt kann das Terpolymer ein Molekulargewicht im Bereich von 500 g/mol bis 100 000 g/mol aufweisen. Besonders bevorzugt kann es ein Molekulargewicht von 5000 g/mol bis 50 000 g/mol aufweisen. Mit diesem Molekulargewicht liegt das erfindungsgemäße Polymer im Bereich der Oligomeren oder kurzkettigen Polymeren. Mit solchen Strukturmerkmalen zeichnet sich das erfindungsgemäße Terpolymer durch eine vorteilhafte Plastizität aus.

Die Glastemperatur des erfindungsgemäßen Terpolymers kann im Bereich von -40 °C bis 20 °C liegen. Bevorzugt kann das Terpolymer eine Glasübergangstemperatur von -30 bis 0 °C aufweisen.

Aufgrund der amorphen Struktur und der niederen Glastemperatur des als wesentlicher Bestandteil enthaltenen Terpolymers ist die erfindungsgemäße Zusammensetzung bei Raumtemperatur leicht plastisch verformbar. Sie zeichnet sich insbesondere dadurch aus, daß sie, insbesondere des Polymer, eine inhärente Viskosität von 0,05 bis 1,3 dl/g, insbesondere von 0,1 bis 0,9 dl/g, gemessen in HFIP bei 30 °C und einer Konzentration von 0,8 Gew.-%, aufweist.

Die erfindungsgemäße Zusammensetzung kann sich mit Vorteil dadurch auszeichnen, daß sie mindestens einen mono- und/oder polyfunktionellen Alkohol, insbesondere in gebundener Form, enthält. In der erfindungsgemäßen Zusammensetzung kann mindestens ein mono- und/oder polyfunktioneller Alkohol in einem Anteil von 0,02 bis 8 Gew.-% enthalten sein.

Die erfindungsgemäße Zusammensetzung kann sich ferner dadurch auszeichnen, daß sie mindestens eine mono- und/oder polyfunktionelle Carbonsäure, deren Anhydride und/oder Ester, insbesondere in gebundener Form, enthält. In der erfindungsgemäßen Zusammensetzung kann mindestens eine mono- und/oder polyfunktionelle Carbonsäure und/oder deren Derivate in einem Anteil von 0,02 bis 8 Gew.-% vorhanden sein.

Durch Zusatz von mindestens einem mono- und/oder polyfunktionellen Alkohol als Molekulargewichtsregler kann das Molekulargewicht der Polymere verringert werden. Ebenso kann durch Zusatz mindestens einer mono- und/oder polyfunktionellen Carbonsäure, deren Anhydrid und/oder Ester als Molekulargewichtsregler das Molekulargewicht der Polymere verringert werden. Molekulargewichtsregler können in einem Anteil von 0,02 bis 8 Gew.-%, insbesondere von 0,5 bis 6 Gew.-% zugesetzt sein.

Als Beispiele für Molekulargewichtsregler vom Alkoholtyp sind zu nennen 1-Dodecanol, Ethylenglykol, Glycerin, 1,6-Hexandiol, Pentaerythritol und Diethylenglykol.

Als Beispiele für Molekulargewichtsregler vom Carbonsäuretyp sind zu nennen mono- und bifunktionelle Carbonsäuren, ihre Anhydride und Ester ausgehend von organischen Säuren wie Adipinsäure, Sebacinsäure und Stearinsäure.

Ferner kann durch Einmischen eines Weichmachers (Plastifizierungsmittel) in das Terpolymer gemäß der Erfindung die Plastizität noch weiter erhöht werden. Bevorzugt sind dabei Weichmacher, die mit dem Terpolymer eine verträgliche Mischung bilden, so daß keine Phasenseparation auftritt. Mit Vorteil kann in der erfindungsgemäßen Zusammensetzung Plastifizierungsmittel in einem Anteil von 1 bis 30 Gew.-% enthalten sein. Beispiele für erfindungsgemäß anwendbare Weichmacher umfassen Fette und Öle (z. B. Rizinusöl), deren Ester und Metallsalze, Glycerin, Diethylphthalate, Polyethylenglykol, Polypropylenglykol, Citrate und Phosphate.

Die Beimischung eines Weichmachers (Blending) kann zur noch heißen Polymerschmelze im direkten Anschluß an die Polymerisationsreaktion erfolgen oder in einem getrennten Verfahrensschritt. Dabei ist die thermische Stabilität des Weichmachers zu berücksichtigen.

Zur Beschleunigung der Knochenneubildung kann der erfindungsgemäße Zusammensetzung mit Vorteil mindestens ein wachstumsförderndes Mittel zugesetzt sein. Als wachstumsfördernde Mittel können anorganische Verbindungen wie Knochensubstanzbausteine und/oder verwandte Verbindungen verwendet sein. Ferner können als wachstumsfördernde Mittel organische Verbindungen zur Knochenwachstumsförderung verwendet sein. Außerdem können Kombinationen von wachstumsfördernden Mitteln zugesetzt sein.

Beispiele für Substanzen, die das Knochenwachstum fördern, umfassen Calciumtriphosphat, Hydroxylapatit und Wachstumsfaktoren wie das "bone morphogenic protein".

Die erfindungsgemäße Zusammensetzung kann sich mit Vorteil dadurch auszeichnen, daß sie mindestens eine Calciumphosphatverbindung, insbesondere Calciumtriphosphat und/oder Hydroxylapatit, in einem Anteil von 3 bis 20 Gew.-% enthält. Insbesondere kann die Zusammensetzung gemäß der Erfindung einen oder mehrere Wachstumsfaktoren enthalten.

Anorganische Substanzen zur Förderung der Knochenregeneration wie Hydroxylapatit und/oder Calciumtriphosphat können aufgrund ihrer Temperaturbeständigkeit nach Abschluß der Polymerisation direkt der noch heißen oder warmen Schmelze des Terpolymers zugegeben werden. Organische Substanzen wie knochenwachstumsfördernde Proteine können aufgrund ihres Denaturierungsverhaltens nur bei Temperaturen unterhalb von 60 °C zugegeben werden. Eine Homogenisierung des Gemischs kann durch Rühren oder Kneten nach den Fachleuten bekannten Verfahren vorgenommen werden.

Mit Vorteil kann zur Verbesserung der Haftung der erfindungsgemäßen Zusammensetzung an Knochengewebe mindestens ein Adhäsionsmittel zugesetzt sein. Gemäß der Erfindung kann Adhäsionsmittel in einem Anteil von 2 bis 30 Gew.-%, insbesondere 5 bis 30 Gew.-%, in der Zusammensetzung enthalten sein. Beispiele für erfindungsgemäß bevorzugte Adhäsionsmittel umfassen Polyvinylpyrrolidone, Zucker, Stärke, Gelatine oder Cellulosederivate. Die Zugabe von Adhäsionsmitteln zur erfindungsgemäßen Zusammensetzung erfolgt nach den Fachleuten bekannten Verfahren.

Die Erfindung umfaßt auch die Verwendung der oben beschriebenen bioresorbierbaren wachsartigen Zusammensetzung als Knochenwachs. Im Rahmen der Erfindung liegt ferner die Verwendung einer bioresorbierbaren wachsartigen Zusammensetzung in Form eines statistischen Terpolymers mit völlig amorpher Struktur, insbesondere wie sie oben beschrieben ist, zur Herstellung eines Knochenwachses, das ganz oder teilweise aus der Zusammensetzung gebildet ist.

Der Abbau der erfindungsgemäßen wachsartigen Zusammensetzung erfolgt im Körper eines Tieres oder eines Menschen durch Hydrolyse, an der Körper- und Gewebeflüssigkeiten beteiligt sind. Durch die Hydrolyse wird die Polymerkette in kleinere und leichter lösliche Fragmente gespalten. Die Bruchstücke werden ggf. unter Beteiligung von Makrophagen weiter abgebaut. Die Abbauprodukte werden durch das Stoffwechselsystem abtransportiert und wie andere Stoffwechselschlacken aus dem Organismus ausgeschieden. Für eine gute Verträglichkeit des resorbierbaren Knochenwachses beim Patienten ist es wichtig, daß sich während des Abbauvorganges keine schädlichen Metaboliten bilden oder anreichern. Polyglykolsäure zeichnet sich insbesondere dadurch aus, daß bei ihrer Zersetzung in vivo keine toxischen Zerfallsprodukte gebildet werden. Die erfindungsgemäß als Comonomere verwendeten Trimethylencarbonat und Caprolacton sind ebenfalls durch gute Verträglichkeit und Vermeidung toxischer Reaktionen gekennzeichnet.

Das Degradationsverhalten des erfindungsgemäßen Terpolymers kann durch Variation des Gesamtglykolidanteils im Polymer verändert werden, da Polyglykolsäure im Vergleich zu Polytrimethylencarbonat und Poly-ε-Caprolacton deutlich geringere Degradationszeiten aufweist.

Ein weiterer Einflußfaktor, durch dessen Variation die Plastizität und das Abbauverhalten des erfindungsgemäßen Polymers im Knochenwachs verändert werden kann, ist die Intensität und Dauer einer etwaigen γ-Bestrahlung. Die Behandlung mit γ-Strahlen kann mit einem teilweisen Molekulargewichtsabbau verbunden sein, der sich in verkürzten Abbauzeiten äußert. Auf diese Weise ist es möglich, die Eigenschaften des Terpolymers gemäß der Erfindung nach den für den Anwendungsfall vorteilhaften Erfordernissen anzupassen. In einer möglichen Ausführungsform der Erfindung kann eine mit Hilfe von γ-Strahlen durchgeführte Sterilisation gleichzeitig zur Steuerung des Degradationsverhaltens dienen.

Die vorliegende Erfindung umfaßt ferner ein Verfahren zur Herstellung einer bioresorbierbaren wachsartigen Zusammensetzung durch statistische Copolymerisation von Glykolid, Trimethylencarbonat und ε-Caprolaction zu einem Terpolymer. Das erfindungsgemäße Verfahren ist dadurch gekennzeichnet, daß das Terpolymer durch statistische Copolymerisation von Trimethylencarbonat, ε-Caprolacton und Glykolid, bei einem Gewichtsanteil von Trimethylencarbonat von 5 bis 70 Gew.-%, bevorzugt 10 bis 40 %, ε-Caprolacton von 5 bis 70 Gew.-%, bevorzugt 10 bis 40 %, und Glykolid von 10 bis 60 Gew.-%, bevorzugt 20 bis 40 %, hergestellt wird.

Die Terpolymerisierungsreaktion kann nach üblichen, den Fachleuten bekannten Verfahrensweisen zur Herstellung von Copolymeren durchgeführt werden. Mit Vorteil wird die Umsetzung als Schmelzpolymerisation bei Temperaturen über 150 °C in einem geeigneten Reaktor durchgeführt, der heizbar und mit einer Rührvorrichtung versehen ist. Der Reaktor ist insbesondere so konzipiert, daß eine gute Durchmischung der Polymerisationsmassen gewährleistet ist, die geforderten Temperaturbereiche eingehalten werden können und das Polymerisat weitgehend vollständig abgelassen werden kann.

In einer bevorzugten Ausführungsform kann die Reaktionsmischung unter stetiger Durchmischung erhitzt werden. Mit Vorteil kann die Polymerisation bei einer Temperatur von 190 bis 210 °C, insbesondere bei 205 °C durchgeführt werden. Für die Dauer der Umsetzung kann ein Überdruck angelegt werden. Mit Vorteil kann die Polymerisation bei einem Überdruck von 1 bis 2 bar Argon, insbesondere 1,5 bar Argon durchgeführt werden. Während der Reaktionsdauer von 2 bis 6 Stunden, bevorzugt 5 Stunden, können sich die vorgelegten Monomere zu einem statistischen Terpolymer umsetzen. Vorteilhaft zeichnet sich das Verfahren dadurch aus, daß die Umsatzrate über 95 % beträgt.

Der Monomerenmischung zur Herstellung des erfindungsgemäßen Polymers kann ein geeigneter Katalysator wie beispielsweise Zinnoctanoat oder Zinn-II-chlorid zugesetzt werden. Ferner kann der Monomerenmischung ein mono- und/oder bifunktioneller Initiator wie beispielsweise 1-Dodecanol, 1,6-Hexandiol oder Diethylenglykol zugesetzt werden.

Mit Vorteil kann bei der Polymerisation mindestens ein Molekulargewichtsregler zugegeben werden. Molekulargewichtsregler kann insbesondere in einem Anteil von 0,02 bis 8 Gew.-% zugegeben werden. Insbesondere kann bei der Polymerisation als Molekulargewichtsregler mindestens ein mono- und/oder polyfunktioneller Alkohol zugegeben werden. In einer Ausführungsform der Erfindung kann der Initiator auch als Molekulargewichtsregler fungieren.

Ferner kann bei der Polymerisation als Molekulargewichtsregler mindestens eine mono- und/oder polyfunktionelle Carbonsäure und/oder deren Derivate zugegeben werden.
Zur Molekulargewichtsregelung können bei Polymerisationsbeginn viele Polymerketten gebildet werden, die entweder an einem Ende keine reaktiven Gruppen zur Fortsetzung der Polymerisierungsreaktion besitzen oder deren beide Enden z. B. hydroxyterminiert sind und somit keine Kettenverlängerung durch Zusammenschluß zweier Kettenmoleküle erfolgen kann.

Im direkten Anschluß an die Polymerisation können zur Schmelze des Terpolymers Additive zur Verbesserung der Eigenschaften zugegeben werden. Als Beispiele für Additive, die zur Verbesserung der Eigenschaften der erfindungsgemäßen Zusammensetzung dem Terpolymer zugesetzt werden können, sind Mittel zur Verbesserung der Plastifizierbarkeit, Mittel zur Verbesserung der Adhäsion an Knochengewebe und/oder Mittel für schnelleres, induziertes Knochenwachstum zu nennen.

In einer Ausführungsform des erfindungsgemäßen Verfahrens kann die Zugabe von Additiven ohne vorheriges Abkühlen der Polymerschmelze erfolgen. Bei dieser Verfahrenweise kann das Additiv bevorzugt unter ständigem Rühren der Polymerschmelze zugegeben werden. In einer anderen Ausführungsform kann die Zugabe von Additiven nach vorheriger Kühlung auf eine tiefere Temperatur erfolgen. Die Wahl der geeigneten Temperatur hängt insbesondere auch von der Temperaturempfindlichkeit des vorgesehenen Additivs ab.

In einer weiteren Ausführungsform der Erfindung kann das Terpolymer aus dem Reaktor abgelassen und nach Erkalten in üblicher Weise zerkleinert und getrocknet werden. Zur Reinigung des Terpolymers können die, wenn auch nur in geringen Mengen vorhandenen Restmonomere, in geeigneter Weise entfernt werden. Beispiele für geeignete Verfahren zur Entfernung von nicht umgesetzten Restmengen an Monomeren umfassen CO₂-Extraktion, Lösemittelextraktion oder Trocknungsprozesse bei erhöhter Temperatur unter Vakuum.

Gemäß der Erfindung kann der wachsartigen Zusammensetzung mindestens ein knochenwachstumsförderndes Protein zugemischt werden. Wie oben erwähnt, können organische Substanzen wie knochenwachstumsfördernde Proteine aufgrund ihres Denaturierungsverhaltens nur bei Temperaturen unterhalb von 60 °C zugegeben werden. Eine homogene Verteilung der Proteine in der Terpolymer enthaltenden erfindungsgemäßen Zusammensetzung kann durch intensive Knetprozesse im Temperaturbereich zwischen 20 bis 45 °C erreicht werden.

Bei einem weiteren vorteilhaften Verfahren zur Inkorporation der wachstumsfördernden Proteine kannn der erfindungsgemäßen Zusammensetzung knochenwachstumsförderndes Protein zugemischt werden, indem das gelöste Terpolymer in einer Lösung des knochenwachstumsfördernden Proteins gefällt wird. Während dieses Präzipitationsvorganges wird das wachstumsfördernde Protein in die Polymerzusammensetzung eingeschlossen. Lösemittel für die Auflösung der Terpolymerzusammensetzung sind so auszuwählen, daß bei Kontakt des Proteins mit dem Lösemittel keine Denaturierung des Proteins auftreten kann.

Die bioresorbierbare Zusammensetzung und die daraus hergestellten medizinischen Produkte gemäß der vorliegenden Erfindung können gefärbt oder ungefärbt sein. Zum Einfärben können für resorbierbare medizinische Vorrichtungen von der amerikanischen FDA (Food and Drug Administration) zugelassene Farbstoffe verwendet werden wie beispielsweise D&C Green Nr. 6, D&C Violet Nr. 2 und andere.

Zur Verwendung als medizinisches Produkt kann die erfindungsgemäße Zusammensetzung in geeigneter Weise sterilisiert werden. Ein zweckmäßiges Sterilisierverfahren kann aus üblichen physikalischen oder chemischen Methoden zum Inaktivieren von Mikroorganismen ausgewählt oder eine Kombination solcher Methoden sein. Ein mögliches Sterilisierungsverfahren umfaßt die Behandlung mit γ-Strahlen. Bevorzugt kann eine Sterilisierung des erfindungsgemäßen Polymermaterials für medizinische Produkte unter Verwendung von Ethylenoxid vorgenommen werden. In einer möglichen Ausführungsform der Erfindung kann eine mit Hilfe von γ-Strahlen durchgeführte Sterilisation gleichzeitig zur Steuerung des Degradationsverhaltens des erfindungsgemäß hergestellten Knochenwachses dienen.

Mit Vorteil kann das aus der erfindungsgemäßen Zusammensetzung hergestellte Knochenwachs in zweckmäßiger Menge portioniert gebrauchsfertig in geeigneter Weise verpackt vorliegen.

Wegen der hydrolytischen Zersetzbarkeit des erfindungsgemäßen Polymermaterials sind die medizinischen Produkte bei ihrer Lagerung vor Feuchtigkeit und erhöhten Temperaturen zu schützen, damit die vorteilhaften Eigenschaften bis zur Verwendung voll erhalten bleiben. Zweckmäßigerweise können sie in eine vor Feuchtigkeit schützende Verpackung verpackt werden, insbesondere einer Verpackung aus feuchtigkeitsundurchlässigem Folienmaterial, bevorzugt einer Vakuumverpackung.

Weitere Merkmale und Einzelheiten der Erfindung ergeben sich aus der nachfolgenden Beschreibung von bevorzugten Ausführungsformen in Form von Beispielen. Dabei können die einzelnen Merkmale jeweils für sich alleine oder zu mehreren in Kombination miteinander verwirklicht sein. Die Beispiele dienen lediglich der Erläuterung der vorliegenden Erfindung, die in keiner Weise darauf beschränkt sein soll.

Bei allen hier beschriebenen Beispielen werden die inhärenten Viskositäten bei 30 °C in Hexafluorisopropanol (HFIP) bei einer Konzentration von 0,8 g/dl gemessen. Die Bestimmung der Glasumwandlungstemperaturen erolgt mittels Differential Scanning Calorimetry (DSC).

### Beispiel 1

### Terpolymer der Zusammensetzung G/TMC/CL = 30/35/35

In einen Reaktor werden 350 g 1,3-Dioxan-2-on (TMC), 350 g caprolacton (CL) und 300 g Glykolid (G) gegeben zusammen mit 0,2 g Zinnoctanoat (Lösung in Diethylether). Der Ether wird danach im Vakuum bei 50 °C abgezogen. Nach einer Stunde wird ein Überdruck von 1,5 bar Argon angelegt und das Reaktionsgemisch unter Rühren auf 205 °C erhitzt. Diese Temperatur wird 5 h aufrechterhalten. Danach wird das Polymer bei einer Temperatur von 180 °C abgelassen und analysiert.

Das Polymer ist transparent, seine inhärente Viskosität beträgt 0,798 dl/g und sein Glasumwandlungspunkt liegt bei -25,6 °C. Das Polymer kann bei Raumtemperatur leicht plastisch verformt werden.

### Beispiel 2

### Terpolymer der Zusammensetzung G/TMC/CL = 30/35/35

Die Polymerisation erfolgt analog zu Beispiel 1 jedoch mit dem Unterschied, daß als Initiator und Molekulargewichtsregler 5 g 1-Dodecanol zur Monomerenmischung zugegeben werden. Das rohe Polymer wird bei 120 °C aus dem Reaktor abgelassen, da es eine niedrigere Schmelzviskosität aufweist als das Produkt von Beispiel 1.

Das Polymer ist transparent, seine inhärente Viskosität beträgt 0,210 dl/g und sein Glasumwandlungspunkt liegt bei -27,3 °C. Die Plastizifierbarkeit dieses Produkts ist gegenüber dem Polymer von Beispiel 1 verbessert.

### Beispiel 3

### Terpolymer der Zusammensetzung G/TMC/CL = 40/30/30

In einen Reaktor werden 300 g 1,3-Dioxan-2-on (TMC), 300 g Caprolacton (CL) und 400 g Glykolid (G) gegeben zusammen mit 0,1 g Zinn-II-chlorid-dihydrat (Lösung in Diethylether). Die Durchführung der Reaktion erfolgt analog zu Beispiel 1.

Das Polymer ist transparent, seine inhärente Viskosität beträgt 0,723 dl/g. Der Glasumwandlungspunkt liegt bei -23,0 °C. Die Plastizifierbarkeit dieses Produkts ist vergleichbar zu dem Terpolymer aus Beispiel 1.

### Beispiel 4

### Terpolymer der Zusammensetzung G/TMC/CL = 40/30/30 mit 7 Gew.-% eines Plastifizierungsmittels bestehend aus Glycerin.

Die Einwaagen der Ausgangsverbindungen und die Reaktionsführung sind analog zu Beispiel 3 mit dem Unterschied, daß nach Beendigung der Reaktion, die Temperatur auf 100 °C erniedrigt und eine Probe des Terpolymers entnommen wird.

Dann werden unter Rühren 70 g Glycerin zugegeben, der Reaktor wieder verschlossen und die Komponenten für weitere 20 Minuten gut durchmischt. Zum Ablassen des Terpolymers wird die Temperatur auf 120 °C erhöht.

Die inhärente Viskosität der vor der Glycerinzugabe entnommenen Probe beträgt 0,698 dl/g, der Glasumwandlungspunkt liegt bei -23,7 °C. Die Plastifizierbarkeit ist gegenüber Beispiel 3 deutlich verbessert.

## Patentansprüche

1. Bioresorbierbare wachsartige Zusammensetzung, dadurch gekennzeichnet, daß sie im wesentlichen aus einem statistischen Terpolymer mit völlig amorpher Struktur gebildet ist.

2. Zusammensetzung nach Anspruch 1, dadurch gekennzeichnet, daß das Terpolymer aus Trimethylencarbonat, ε-Caprolacton und Glykolid gebildet ist.

3. Zusammensetzung nach einem der vorhergehenden Ansprüche, dadurch gekennzeichnet, daß im Terpolymer Trimethylencarbonat in einem Anteil von 5 bis 70 Gew.-%, ε-Caprolacton in einem Anteil von 5 bis 70 Gew.-% und Glykolid in einem Anteil von 10 bis 60 Gew.-% enthalten ist.

4. Zusammensetzung nach einem der vorhergehenden Ansprüche, dadurch gekennzeichnet, daß im Terpolymer Trimethylencarbonat und ε-Caprolacton in einem Gewichtsverhältnis zwischen 80 : 20 und 20 : 80 vorhanden sind.

5. Zusammensetzung nach einem der vorhergehenden Ansprüche, dadurch gekennzeichnet, daß das Terpolymer durch statistische Copolymerisation von Trimethylencarbonat, ε-Caprolacton und Glykolid hergestellt ist.

6. Zusammensetzung nach einem der vorhergehenden Ansprüche, dadurch gekennzeichnet, daß das Terpolymer ein Molekulargewicht im Bereich von 500 g/mol bis 100000 g/mol aufweist.

7. Zusammensetzung nach einem der vorhergehenden Ansprüche, dadurch gekennzeichnet, daß sie, insbesondere das Polymer, eine inhärente Viskosität von 0,05 bis 1,3 dl/g, insbesondere von 0,1 bis 0,9 dl/g, gemessen in HFIP bei 30 °C und einer Konzentration von 0,8 Gew.-%, aufweist.

8. Zusammensetzung nach einem der vorhergehenden Ansprüche, dadurch gekennzeichnet, daß sie mindestens einen mono- und/oder polyfunktionellen Alkohol, insbesondere in gebundener Form, enthält.

9. Zusammensetzung nach einem der vorhergehenden Ansprüche, dadurch gekennzeichnet, daß sie mindestens eine mono- und/oder polyfunktionelle Carbonsäure, deren Anhydride und/oder Ester, insbesondere in gebundener Form, enthält.

10. Zusammensetzung nach einem der vorhergehenden Ansprüche, dadurch gekennzeichnet, daß sie mindestens ein Plastifizierungsmittel in einem Anteil von 1 bis 30 Gew.-% enthält.

11. Zusammensetzung nach einem der vorhergehenden Ansprüche, dadurch gekennzeichnet, daß sie mindestens eine Calciumphosphatverbindung, insbesondere Tricalciumphosphat und/oder Hydroxylapatit, in einem Anteil von 3 bis 20 Gew.-% enthält.

12. Zusammensetzung nach einem der vorhergehenden Ansprüche, dadurch gekennzeichnet, daß sie einen oder mehrere Wachstumsfaktoren enthält.

13. Zusammensetzung nach einem der vorhergehenden Ansprüche, dadurch gekennzeichnet, daß sie mindestens ein Adhäsionsmittel in einem Anteil von 2 bis 30 Gew.-% enthält.

14. Verwendung der Zusammensetzung nach einem der vorhergehenden Ansprüche als Knochenwachs.

15. Verwendung einer bioresorbierbaren wachsartigen Zusammensetzung in Form eines statistischen Terpolymers mit völlig amorpher Struktur, insbesondere nach einem der vorhergehenden Ansprüche, zur Herstellung eines Knochenwachses, das ganz oder teilweise aus der Zusammensetzung gebildet ist.

16. Verfahren zur Herstellung einer bioresorbierbaren wachsartigen Zusammensetzung, insbesondere nach einem der vorhergehenden Ansprüche, dadurch gekennzeichnet, daß ein Terpolymer durch statistische Copolymerisation von Trimethylencarbonat, ε-Caprolacton und Glykolid, bei einem Gewichtsanteil von Trimethylencarbonat von 5 bis 70 Gew.-%, bevorzugt 10 bis 40 %, ε-Caprolacton von 5 bis 70 Gew.-%, bevorzugt 10 bis 40 %, und Glykolid von 10 bis 60 Gew.-%, bevorzugt 20 bis 40 %, hergestellt wird.

17. Verfahren nach Anspruch 16, dadurch gekennzeichnet, daß bei der Polymerisation mindestens ein Molekulargewichtsregler in einem Anteil von 0,02 bis 8 Gew.-% zugegeben wird.

18. Verfahren nach Anspruch 16 oder 17, dadurch gekennzeichnet, daß bei der Polymerisation als Molekulargewichtsregler mindestens ein mono- und/oder polyfunktioneller Alkohol zugegeben wird.

19. Verfahren nach einem der Ansprüche 16 bis 18, dadurch gekennzeichnet, daß bei der Polymerisation als Molekulargewichtsregler mindestens eine mono- und/oder polyfunktionelle Carbonsäure und/oder deren Derivate zugegeben wird.

20. Verfahren nach einem der Ansprüche 16 bis 19, dadurch gekennzeichnet, daß der Zusammensetzung mindestens ein knochenwachstumsförderndes Protein zugemischt wird.
